# EUROPÄISCHE PATENTANMELDUNG

(11) **EP 4 254 420 A1**
(43) Veröffentlichungstag der Anmeldung: **04.10.2023**
(21) Anmeldenummer: 22165902.2
(22) Anmeldetag: 31.03.2022
(51) Int. Cl.: G16C 20/10, G05B 13/04, G05B 19/00

(54) **VERFAHRENSTECHNISCHES ANLAGENMODUL UND VERFAHREN ZUM STEUERN EINES VERFAHRENSTECHNISCHEN ANLAGENMODULS**

(71) Anmelder: Siemens Aktiengesellschaft, 80333 München (DE)
(72) Erfinder: Baudisch, Thomas, 86938 Schondorf am Ammersee (DE); Haager, Dominik, 83052 Bruckmühl (DE); Held, Harald, 85461 Bockhorn (DE); Jastram, Alexander, 80637 München (DE); Kunzelmann, Robert Niklas, 81369 München (DE); Wehrstedt, Jan Christoph, 81829 München (DE); Wincheringer, Christoph, 81369 München (DE)
(74) Vertreter: Siemens Patent Attorneys

(57) **Zusammenfassung**

Die Erfindung betrifft ein verfahrenstechnisches Anlagenmodul (AM) umfassend:
a. Anlagenkomponenten (K), die dazu eingerichtet sind, einen verfahrenstechnischen Prozess durchzuführen, wobei ein erster chemischer Stoff (CS1) in dem verfahrenstechnischen Prozess geändert wird,
b. einen Sensor (FM), der derart eingerichtet ist, einen Sensorwert einer physikalischen Größe (Q) des ersten chemischen Stoffs in einer Anlagenkomponente zu erfassen,
c. eine Simulationseinheit (SIM), die derart eingerichtet ist,
- eine chemische Reaktion, eine Temperaturänderung und/oder einen Stofftransport des verfahrenstechnischen Prozesses für einen zweiten chemischen Stoff (CS2) in Abhängigkeit des Sensorwerts (Q) computergestützt zu simulieren, wobei der erste chemische Stoff und der zweite chemische Stoff übereinstimmen oder sich unterscheiden, und
- einen simulierten Temperaturwert (T) und einen simulierten Stoffmengenanteil (x) des zweiten chemischen Stoffs auszugeben,
und
d. eine Steuereinheit (PLC), die derart eingerichtet ist, Aktoren der Anlagenkomponenten in Abhängigkeit des simulierten Temperaturwerts, des simulierten Stoffmengenanteils und des erfassten Sensorwerts zu steuern.

Die Erfindung ermöglicht insbesondere eine hybride Inbetriebnahme einer verfahrenstechnischen Anlage, bei der parallel zu einer Wasserfahrt eine computergestützte Simulation des verfahrenstechnischen Prozesses durchgeführt wird.

## Beschreibung

Die Erfindung betrifft ein verfahrenstechnisches Anlagenmodul und ein Verfahren zum Steuern von Anlagenkomponenten eines verfahrenstechnischen Anlagenmoduls mittels einer computergestützten Simulation des verfahrenstechnischen Prozesses, sowie ein Computerprogrammprodukt.

Vor einem produktiven Einsatz von verfahrenstechnischen Anlagen werden diese in Betrieb genommen, um ihre Funktionalität zu testen und Fehler zu vermeiden. Hierbei gibt es die virtuelle Inbetriebnahme, bei der eine Automatisierung der Anlage mittels einer computergestützten Simulation des technischen Prozesses validiert wird. In der Regel wird dabei eine sogenannte Wasserfahrt simuliert. Eine Wasserfahrt bezeichnet hierbei eine Funktionsprüfung auf Basis von Wasser oder anderen chemisch inerten Stoffen, anstatt der eigentlich vorgesehenen chemischen Stoffe des Prozesses. Der technische Prozess wird in der Regel nicht simuliert, um die Echtzeitfähigkeit der Simulation zu erhalten und die Komplexität der Modellierung gering zu halten. Anschließend findet eine reale Inbetriebnahme statt. Dabei wird in der Regel auch eine Wasserfahrt in der realen Anlage betrachtet, d.h. anstatt der realen Medien wird die Anlage mit ungefährlichen inerten Stoffen getestet. Somit können die Durchflussregelungen und anderen Funktionalitäten der Anlage konfiguriert und validiert werden, bevor man auf die realen Betriebsbedingungen und Stoffmedien wechselt. Allerdings erfolgt somit die Inbetriebnahme der verfahrenstechnischen Anlage nicht unter Realbedingungen, d.h. insbesondere die chemischen Reaktionen werden nicht berücksichtigt und so kann die Anlage nicht mit den vorgesehenen chemischen Stoffen vorab getestet werden.

Es ist daher eine Aufgabe der Erfindung, eine Inbetriebnahme einer verfahrenstechnischen Anlage zu ermöglichen, bei der auch der verfahrenstechnische Prozess validiert wird.

Die Aufgabe wird durch die in den unabhängigen Ansprüchen beschriebenen Maßnahmen gelöst. In den abhängigen Ansprüchen sind vorteilhafte Weiterbildungen der Erfindung dargestellt.

Gemäß einem ersten Aspekt betrifft die Erfindung ein verfahrenstechnisches Anlagenmodul umfassend:
a. Anlagenkomponenten, die dazu eingerichtet sind, einen verfahrenstechnischen Prozess durchzuführen, wobei ein erster chemischer Stoff in dem verfahrenstechnischen Prozess geändert wird,
b. mindestens einen Sensor, der derart eingerichtet ist, einen Sensorwert einer physikalischen Größe des ersten chemischen Stoffs in einer Anlagenkomponente zu erfassen,
c. eine Simulationseinheit, die derart eingerichtet ist,
   - eine chemische Reaktion, eine Temperaturänderung und/oder einen Stofftransport des verfahrenstechnischen Prozesses für einen zweiten chemischen Stoff in Abhängigkeit des Sensorwerts computergestützt zu simulieren, wobei der erste chemische Stoff und der zweite chemische Stoff übereinstimmen oder sich unterscheiden,
      und
   - einen simulierten Temperaturwert und einen simulierten Stoffmengenanteil des zweiten chemischen Stoffs auszugeben,
      und
d. eine Steuereinheit, die derart eingerichtet ist, Aktoren der Anlagenkomponenten in Abhängigkeit des simulierten Temperaturwerts, des simulierten Stoffmengenanteils und des erfassten Sensorwerts zu steuern.

Gemäß einem zweiten Aspekt betrifft die Erfindung ein Verfahren zum Steuern von Anlagenkomponenten eines verfahrenstechnischen Anlagenmoduls, mit den Verfahrensschritten:
a. Erfassen eines Sensorwerts einer physikalischen Größe eines ersten chemischen Stoffs in einer Anlagenkomponenten des verfahrenstechnischen Anlagenmoduls, wobei der erste chemische Stoff in einem in den Anlagenkomponenten des verfahrenstechnischen Anlagenmoduls ablaufenden verfahrenstechnischen Prozess geändert wird,
b. computergestütztes Simulieren einer chemischen Reaktion, einer Temperaturänderung und/oder eines Stofftransports des verfahrenstechnischen Prozesses für einen zweiten chemischen Stoff in Abhängigkeit des Durchflusswerts, wobei der erste chemische Stoff und der zweite chemische Stoff übereinstimmen oder sich unterscheiden,
c. Ausgeben eines simulierten Temperaturwerts und eines simulierten Stoffmengenanteils des zweiten chemischen Stoffs, und
d. Steuern von Aktoren der Anlagenkomponenten in Abhängigkeit des simulierten Temperaturwerts, des simulierten Stoffmengenanteils und des erfassten Sensorwerts.

Das verfahrenstechnische Anlagenmodul kann insbesondere auch als ein prozesstechnisches Anlagenmodul oder als Anlagenmodul der Prozessindustrie bezeichnet werden. Ein verfahrenstechnisches Anlagenmodul kann mit mindestens einem weiteren verfahrenstechnischen Anlagenmodul zu einer verfahrenstechnischen Anlage gekoppelt werden. Ein verfahrenstechnisches Anlagenmodul kann aber auch selbst als eine verfahrenstechnische Anlage umfassend eine Vielzahl von Anlagenkomponenten, auch als Anlagenmodule bezeichnet, angesehen werden. In anderen Worten, eine verfahrenstechnische Anlage kann ebenso als Anlagenmodul betrachtet werden.

Im verfahrenstechnischen Anlagenmodul kann ein verfahrenstechnischer Prozess ablaufen, bei dem ein chemischer Stoff, d.h. ein Element, eine Verbindung oder ein Gemisch, der als Flüssigkeit, Feststoff oder Gas vorliegen kann, geändert wird. Unter einer Änderung eines chemischen Stoffs in einem verfahrenstechnischen Prozess kann eine Stoffumwandlung, d.h., eine Änderung des chemischen Stoffs hinsichtlich Zusammensetzung, Art oder Eigenschaften verstanden werden. Im Falle eine Wasserfahrt, z.B. bei einer Inbetriebnahme einer verfahrenstechnischen Anlage, ist der chemische Stoff in der Regel Wasser oder Stickstoff im Falle von Gasen.

Eine computergestützte Simulation des verfahrenstechnischen Prozesses wird mittels eines computergestützten Simulationsmodells des verfahrenstechnischen Prozesses durchgeführt, wobei das Simulationsmodell dazu eingerichtet ist, mindestens eine chemische Reaktion, eine Mischung, eine Temperaturänderung und/oder einen Stofftransport des verfahrenstechnischen Prozesses abzubilden. Dabei werden insbesondere der mindestens eine chemische Stoff betrachtet, der nach einer Inbetriebnahme des verfahrenstechnischen Anlagenmoduls/der verfahrenstechnischen Anlage dafür vorgesehen ist. Das Simulationsmodell kann auch als digitaler Zwilling des verfahrenstechnischen Prozesses bezeichnet werden.

Die Erfindung ermöglicht insbesondere eine hybride Inbetriebnahme einer verfahrenstechnischen Anlage, bei der parallel zu einer Wasserfahrt eine computergestützte Simulation des verfahrenstechnischen Prozesses bzw. mindestens eines Verfahrensschritts oder Aspekts des verfahrenstechnischen Prozesses durchgeführt wird. Die Simulationsergebnisse können dann zum Steuern der Anlage genutzt werden. Eine hybride Inbetriebnahme bedeutet, dass ein Teil der Verfahrensschritte in der realen Anlage während der Wasserfahrt durch eine Simulation ersetzt werden kann. In anderen Worten, bei einer Inbetriebnahme kann ein simulierter Sensorwert anstelle eines realen Sensorwerts zum Steuern des Anlagenmoduls genommen werden. Vorzugsweise wird lediglich ein Prozessschritt virtualisiert, der für die Inbetriebnahme des Anlagenmoduls wichtig ist. Dazu wird das Anlagenmodul vorzugsweise so eingerichtet, dass es ein Edge-Gerät umfasst, auf welchem Schritte des verfahrenstechnischen Prozesses parallel zu Wasserfahrt simuliert werden können. So kann eine Automatisierung mit Signalen aus der Simulation ergänzt werden, um die Steuerung des Anlagenmoduls unter realistischen Bedingungen zu validieren. Beispielweise kann ein Durchflussregler, der die Stoffmengenkonzentration eines Eduktes in einen Reaktionsbehälter konstant halten soll, schon während der Wasserfahrt in der realen Anlage validiert werden, indem die Reaktion, die Temperaturänderung, die Mischung von chemischen Stoffen und/oder die Stoffmengenänderung im Reaktor simuliert werden, obwohl diese Reaktion bei der Wasserfahrt in der Realität noch nicht stattfindet.

Außerdem ermöglicht die Erfindung, ein verfahrenstechnisches Anlagenmodul zu betreiben, wobei Werte aus der Simulation für die Steuerung herangezogen werden können. Beispielsweise können virtuelle Sensorwerte für die Steuerung des verfahrenstechnischen Anlagenmoduls genutzt werden. Damit kann die Genauigkeit der Steuerung und/oder die Betriebssicherheit verbessert werden.

In einer Ausführungsform der Erfindung kann der erste chemische Stoff chemisch inert sein.

Insbesondere kann der erste chemische Stoff Wasser und/oder Stickstoff sein. So kann in den realen Anlagenkomponenten eine Wasserfahrt durchgeführt werden, um beispielsweise mechanische Funktionalitäten des Anlagenmoduls zu testen.

In einer Ausführungsform der Erfindung kann der Sensor als virtueller Sensor ausgestaltet sein und einen simulierten Sensorwert bereitstellen.

Somit können zusätzlich oder alternativ simulierte Sensorwerte erfasst und für die Steuerung genutzt werden.

In einer Ausführungsform der Erfindung kann der Sensor ein Durchflusssensor oder ein Füllstandsensor sein.

In einer Ausführungsform der Erfindung können Steuerbefehle der Steuereinheit bei der computergestützten Simulation des verfahrenstechnischen Prozesses berücksichtigt werden.

Dies bedeutet, dass die Simulation mittels der aktuellen Steuerbefehle angepasst werden kann. Dies ermöglicht eine parallel zur realen Wasserfahrt durchgeführte Simulation.

In einer Ausführungsform der Erfindung kann die computergestützte Simulation in Echtzeit und parallel zum verfahrenstechnischen Prozess durchgeführt werden.

Gemäß einem weiteren Aspekt betrifft die Erfindung eine verfahrenstechnische Anlage umfassend mindestens zwei erfindungsgemäße verfahrenstechnische Anlagenmodule sowie eine übergeordnete Steuereinheit, wobei die jeweiligen Steuereinheiten der verfahrenstechnischen Anlagenmodule durch die übergeordnete Steuereinheit miteinander gekoppelt werden und wobei die jeweiligen Simulationseinheiten der verfahrenstechnischen Anlagenmodule miteinander gekoppelt werden.

Alternativ kann die verfahrenstechnische Anlage lediglich eine Simulationseinheit umfassen, die als Edge-Gerät oder in der Cloud realisiert sein kann.

In einer Ausführungsform der verfahrenstechnischen Anlage können die jeweiligen Simulationseinheiten der verfahrenstechnischen Anlagenmodule mittels einer Co-Simulationsumgebung miteinander gekoppelt werden und ein von der ersten Simulationseinheit ausgegebener erster simulierter Temperaturwert und ein erster simulierter Stoffmengenanteil als Eingabedaten können für die zweite Simulationseinheit genutzt werden.

Des Weiteren betrifft die Erfindung ein Computerprogrammprodukt, das direkt in einen programmierbaren Computer ladbar ist, umfassend Programmcodeteile, die bei der Ausführung des Programms durch einen Computer diesen veranlassen, die Schritte eines erfindungsgemäßen Verfahrens auszuführen.

Ein Computerprogrammprodukt kann beispielsweise auf einem Speichermedium, wie z.B. Speicherkarte, USB-Stick, CD-ROM, DVD, ein nichtflüchtiger/dauerhaftes Speichermedium (engl. Non-transitory storage Medium) oder auch in Form einer herunterladbaren Datei von einem Server in einem Netzwerk bereitgestellt oder geliefert werden.

Ausführungsbeispiele der Erfindung sind in den Zeichnungen beispielhaft dargestellt und werden anhand der nachfolgenden Beschreibung näher erläutert. Es zeigen:
- Fig. 1:: ein Ausführungsbeispiel eines verfahrenstechnischen Anlagenmoduls;
- Fig. 2:: ein Ausführungsbeispiel einer verfahrenstechnischen Anlage;
- Fig. 3:: ein erstes Ausführungsbeispiel eines Verfahrens zum Steuern von Anlagenkomponenten eines verfahrenstechnischen Anlagenmoduls; und
- Fig. 4:: ein zweites Ausführungsbeispiel eines Verfahrens zum Steuern von Anlagenkomponenten eines verfahrenstechnischen Anlagenmoduls.

Einander entsprechende Teile sind in allen Figuren mit den gleichen Bezugszeichen versehen.

Insbesondere zeigen die nachfolgenden Ausführungsbeispiele lediglich beispielhafte Realisierungsmöglichkeiten, wie insbesondere solche Realisierungen der erfindungsgemäßen Lehre aussehen könnten, da es unmöglich und auch für das Verständnis der Erfindung nicht zielführend oder notwendig ist, all diese Realisierungsmöglichkeiten zu benennen.

Auch sind insbesondere einem (einschlägigen) Fachmann in Kenntnis des/der Verfahrensanspruchs/Verfahrensansprüche alle im Stand der Technik üblichen Möglichkeiten zur Realisierung von Produkten oder Möglichkeiten zur Implementierung selbstverständlich bekannt, sodass es insbesondere einer eigenständigen Offenbarung in der Beschreibung nicht bedarf. Insbesondere können diese gebräuchlichen und dem Fachmann bekannten Realisierungsvarianten ausschließlich per Hardware(komponenten) oder ausschließlich per Software(komponenten) realisiert werden. Alternativ und/oder zusätzlich kann der Fachmann im Rahmen seines fachmännischen Könnens weitestgehend beliebige erfindungsgemäße Kombinationen aus Hardware(komponenten) und Software(komponenten) wählen, um erfindungsgemäße Realisierungsvarianten umzusetzen.

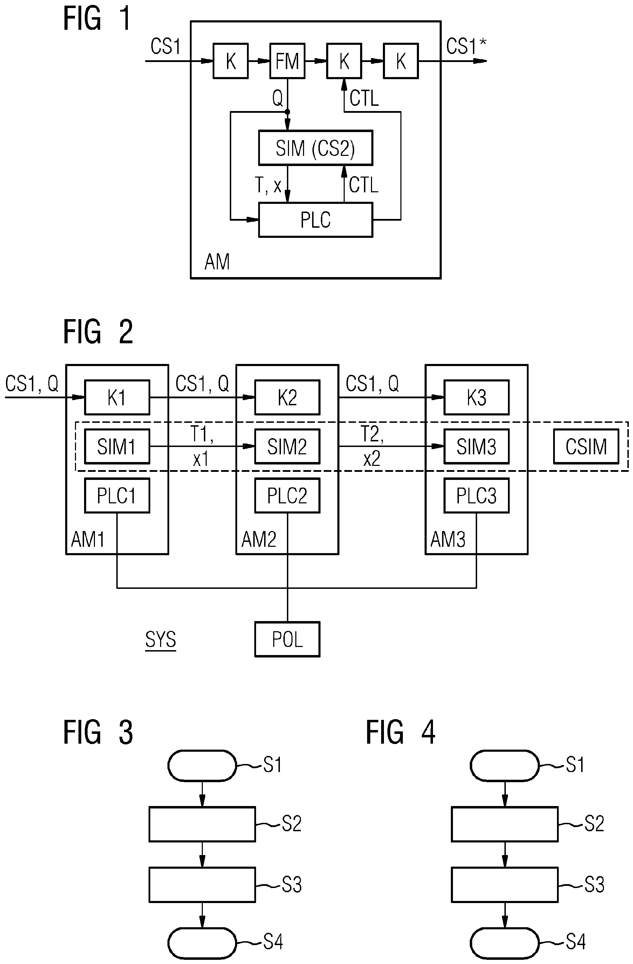

Figur 1 zeigt ein Ausführungsbeispiel eines erfindungsgemäßen verfahrenstechnischen Anlagenmoduls AM. Mit dem verfahrenstechnischen Anlagenmodul kann insbesondere eine hybride virtuelle Inbetriebnahme realisiert werden, wobei eine Wasserfahrt mit einer computergestützten Simulation der chemischen Reaktion, die Temperaturänderung, die Vermischung von Stoffen und/oder des Stofftransports kombiniert werden. Dafür ist das verfahrenstechnische Anlagenmodul AM vorzugsweise mit einem Edge-Gerät ausgestattet, auf dem parallel zur realen Wasserfahrt der verfahrenstechnische Prozess simuliert werden kann. Alternativ kann die computergestützte Simulation auch auf der Cloud durchgeführt werden. Somit können beispielsweise nicht nur Durchflüsse, sondern auch Stofftransport, Temperaturänderung und chemische Reaktionen während der Wasserfahrt betrachtet und validiert werden.

Das verfahrenstechnische Anlagenmodul AM umfasst insbesondere Software- und Hardwarekomponenten. Das verfahrenstechnische Anlagenmodul AM umfasst eine Vielzahl von (Hardware-)Anlagenkomponenten K, wie z.B. chemische Reaktoren, welche über Leitungen oder Rohre (nicht dargestellt) miteinander verbunden sind, so dass ein Stofftransport zwischen den Anlagenkomponenten K realisiert werden kann. Das verfahrenstechnische Anlagenmodul AM ist also derart eingerichtet, einen verfahrenstechnischen Prozess und/oder eine Wasserfahrt durchzuführen, wobei ein erster chemischer Stoff CS1 geändert wird. Der verfahrenstechnische Prozess kann beispielsweise eine Eigenschaft des ersten chemischen Stoffs CS1 ändern, wie z.B. eine Temperaturänderung, so dass ein geänderter chemischer Stoff CS1* ausgegeben wird.

Das verfahrenstechnische Anlagemodul AM umfasst weiter mindestens einen Sensor FM, wie z.B. Durchflussmesser oder einen Füllstandsensor, eine Simulationseinheit SIM und eine Steuereinheit PLC. Der Sensor FM kann insbesondere auch außerhalb des verfahrenstechnischen Anlagenmoduls AM, beispielsweise in einem gekoppelten Anlagenmodul, lokalisiert sein oder der Sensor kann mit dem verfahrenstechnischen Anlagenmodul AM lediglich gekoppelt sein, wobei Sensordaten über eine Eingabeeinheit des verfahrenstechnischen Anlagenmoduls bereitgestellt werden.

Die Steuereinheit PLC ist insbesondere die physikalische Steuereinheit des Anlagenmoduls AM, d.h. die Steuereinheit steuert die Aktoren der Anlagenkomponenten K an. Die Steuereinheit ist vorzugsweise eine speicherprogrammierbare Steuerung (SPS; engl. programmable logic controller, PLC). In der Architektur sind die Aktor- und Sensorgrößen, die den Stofftransportprozess bedienen, vorzugsweise direkt mit den realen Sensoren verbunden. Die Signale wie Konzentrationen, Temperatur etc. kommen vorzugsweise aus der Simulation.

Der mindestens eine Sensor FM ist derart eingerichtet, einen Sensorwert einer physikalischen Größe des ersten chemischen Stoffs CS1, wie z.B. Durchfluss, Temperatur, Dichte, Druck etc., zu messen. Der Sensor FM kann beispielsweise in einer Anlagenkomponente, beim Eingang und/oder beim Ausgang des verfahrenstechnischen Anlagenmoduls AM oder einer Anlagenkomponente K oder zwischen zwei Anlagenkomponenten K platziert sein. Beispielsweise ist der Sensor ein Durchflussmesser FM, der einen Durchflusswert Q des ersten chemischen Stoffs CS1 in einer Anlagenkomponente erfasst. Alternativ kann der Sensor auch ein Füllstandsensor sein, der einen Füllstand des ersten chemischen Stoffs CS1 in einer Anlagenkomponente K misst.

Die Simulationseinheit SIM umfasst insbesondere auch einen physikalischen Rechner/Prozessor. Beispielsweise kann die Simulationseinheit SIM ein Edge-Gerät umfassen, auf welchem eine computergestützte Simulation einer chemischen Reaktion, einer Temperaturänderung und/oder eines Stofftransports des verfahrenstechnischen Prozesses ausgeführt wird. Alternativ kann die computergestützte Simulation auch in der Cloud durchgeführt werden, wobei die Simulationsdaten der Simulationseinheit bereitgestellt werden.

Die Simulationseinheit SIM umfasst vorzugsweise mindestens ein computergestütztes Simulationsmodell des verfahrenstechnischen Prozesses. Die Simulationseinheit SIM ist dazu eingerichtet, Teile des verfahrenstechnischen Prozesses für mindestens einen zweiten chemischen Stoff CS2 in Abhängigkeit des vom Durchflusssensor FM erfassten Durchflusswerts Q computergestützt mittels des Simulationsmodells zu simulieren und einen simulierten Temperaturwert T und einen simulierten Stoffmengenanteil x des zweiten chemischen Stoffs CS2 als Simulationsergebnis auszugeben. Der erste chemische Stoff C1 kann sich dabei vom zweiten chemischen Stoff CS2 unterscheiden oder mit diesem übereinstimmen. Insbesondere kann die Simulation für mehr als einen zweiten chemischen Stoff durchgeführt werden.

In einem ersten Fall kann es sich beispielsweise um eine Inbetriebnahme des verfahrenstechnischen Anlagenmoduls handeln, wobei der erste chemische Stoff CS1 sich vom zweiten chemischen Stoff CS2 unterscheidet. Dabei kann der erste chemische Stoff CS1 beispielsweise ein chemisch inerter Stoff, wie z.B. Wasser oder Stickstoff, sein. Beispielsweise wird in den Anlagenkomponenten K, gesteuert durch die Steuereinheit PLC, eine Wasserfahrt durchgeführt. Bei der Wasserfahrt wird der erste chemische Stoff CS1 anstatt des zweiten chemischen Stoffs CS2 von den Anlagenkomponenten prozessiert, wobei insbesondere keine chemische Reaktion stattfindet. Es wird somit eine Funktionsprüfung des Anlagenmoduls AM durchgeführt. Der zweite chemische Stoff CS2 kann beispielsweise derjenige chemische Stoff sein, der nach Inbetriebnahme des verfahrenstechnischen Anlagenmoduls dafür vorgesehen ist. Mittels einer computergestützten Simulation von Teilen des verfahrenstechnischen Prozesses kann beispielsweise eine chemische Reaktion, eine Temperaturänderung und/oder ein Stofftransport des zweiten chemischen Stoffs CS2 (oder mehrerer zweiter chemischen Stoffe) parallel zu einer real durchgeführten Wasserfahrt simuliert werden. Dabei werden Steuerbefehle der Steuereinheit bei der Simulation berücksichtigt. Die computergestützte Simulation liefert einen Temperaturwert T und Stoffmengenwert x des zweiten chemischen Stoffs CS2 zu einem bestimmten Zeitpunkt. Der simulierten Temperaturwerte T und Stoffmengenwert x werden an die Steuereinheit PLC des Anlagenmoduls AM übermittelt und bei der Steuerung des Anlagenmoduls berücksichtigt. Die Steuereinheit PLC steuert Aktoren der Anlagenkomponenten K des Anlagenmoduls in Abhängigkeit des erfassten Durchflusswerts Q, des simulierten Temperaturwerts T und des Stoffmengenwerts x. Insbesondere werden die simulierten Werte anstatt der realen Werte der Wasserfahrt für die Inbetriebnahme genutzt. Somit sind also die Aktor- und Sensorgrößen, die den Wassertransportprozess bedienen, direkt mit den realen Sensoren verbunden. Die Signale wie Konzentrationen, Temperatur etc. kommen aus dem Simulationsmodell. Ein Datenaustausch zwischen der Simulationseinheit SIM und der Steuereinheit PLC kann beispielsweise über OPC UA erfolgen. Der Austausch der realen und simulierten Sensorwerte Q, T, x richten sich dabei vorzugsweise nach einer vorgegebenen Abtastrate der Steuereinheit PLC.

Im zweiten, alternativen Fall kann es sich beispielsweise um einen Betrieb des verfahrenstechnischen Moduls AM handeln, wobei der zweite chemische Stoff CS2 dem ersten chemischen Stoff CS1 entspricht. Vorzugsweise handelt es sich hierbei um den chemischen Stoff, der für das Anlagenmodul AM vorgesehen ist. Während eines Betriebs des verfahrenstechnischen Moduls AM kann somit zumindest ein Teil des verfahrenstechnischen Prozesses parallel computergestützt simuliert werden. Der simulierte Temperaturwert T und der simulierte Stoffmengenanteil x des prozessierten chemischen Stoffs können anstatt entsprechender, real gemessener Werte für die Steuerung PLC des verfahrenstechnischen Anlagenmoduls AM genutzt werden. Zusätzlich kann mittels der computergestützten Simulation auch ein simulierter Durchflusswert ermittelt und für die Steuerung PLC genutzt werden, d.h. der Durchflusssensor kann als virtueller Sensor realisiert werden.

Figur 2 zeigt ein Ausführungsbeispiel einer verfahrenstechnischen Anlage SYS.

Die verfahrenstechnische Anlage SYS umfasst mindestens zwei, bevorzugt eine Vielzahl von verfahrenstechnischen Anlagenmodulen AM1-AM3, wie beispielhaft in Figur 1 dargestellt. Die verfahrenstechnischen Anlagenmodule AM1-AM3 bzw. deren Anlagenkomponenten K1-K3 sind durch Leitungen oder Rohre (nicht dargestellt) miteinander gekoppelt, um einen verfahrenstechnischen Prozess auszuführen.

Die verfahrenstechnische Anlage SYS umfasst weiter eine übergeordnete Steuereinheit POL, auch als Orchestrierungseinheit/Orchestrierungsebene (oder ProcessOrchestrationLayer (POL)) bezeichnet, die die jeweiligen Steuereinheit PLC1-PLC3 der verfahrenstechnischen Anlagenmodule AM1-AM3 miteinander koppelt. So kann eine gekoppelte Steuerung realisiert werden.

Die jeweiligen Simulationseinheiten SIM1-SIM3 der verfahrenstechnischen Anlagenmodule AM1-AM3 sind miteinander gekoppelt, um Simulationsdaten auszutauschen. Vorzugsweise sind die Simulationseinheiten SIM1-SIM3 mittels einer Co-Simulationsumgebung CSIM miteinander gekoppelt. Alternativ kann die verfahrenstechnische Anlage SYS lediglich eine Simulationseinheit umfassen.

In den Anlagenmodulen AM1-AM3 wird ein verfahrenstechnischer Prozess eines chemischen Stoffs CS1 durchgeführt. Der chemische Stoff CS1 wird in den jeweiligen Anlagenkomponenten K1-K3 der Anlagenmodule AM1-AM3 prozessiert. Beispielsweise wird bei einer Inbetriebnahme der verfahrenstechnischen Anlage SYS eine Wasserfahrt durchgeführt. Parallel zum (realen) Ablauf des verfahrenstechnischen Prozesses bzw. der Wasserfahrt wird der verfahrenstechnische Prozess eines jeweiligen Anlagenmoduls AM1-AM3 in den entsprechenden Simulationseinheiten SIM1-SIM3 simuliert. Jeweilige Simulationsergebnisse der jeweiligen Anlagenmodule AM1-AM3 werden als Eingabedaten für das nachfolgende Anlagenmodul genutzt. Beispielsweise wird ein simulierter Temperaturwert T1 und ein simulierter Stoffmengenwert ×1 als Ausgabe der ersten Simulationseinheit SIM1 als Eingabe für die zweite Simulationseinheit SIM2 genutzt. Entsprechend wird ein simulierter Temperaturwert T2 und ein simulierter Stoffmengenwert x2 als Ausgabe der zweiten Simulationseinheit SIM2 als Eingabe für die dritte Simulationseinheit SIM3 genutzt etc.

Auf diese Weise kann eine verfahrenstechnische Anlage aus mehreren Anlagenmodulen AM1-AM3 in Betrieb gesetzt bzw. betrieben werden, wobei die physikalischen Verbindungen zwischen den Anlagenmodulen (Wasser, Druckluft etc.) miteinander verbunden werden und die Steuerungen über eine Steuerung der Prozessführungsebene POL gekoppelt werden. Die virtuellen Werte des digitalen Zwillings des verfahrenstechnischen Prozesses wie Stoffe, Temperaturen und ggf. Dichten werden mittels eines Co-Simulationsansatzes über die Anlagenmodule AM1-AM3 gekoppelt. Diese Kommunikation der Kopplung zwischen Modulen in der Co-simulation braucht insbesondere weniger performant zu sein als die Kopplung zwischen der Simulation und den Prozessgrößen in jedem einzelnen Modul, da der Materialtransport zwischen Modulen durch Rohre träger ist als die chemischen Reaktionen und deren Kopplung mit der Automatisierung.

Figur 3 zeigt ein Ausführungsbeispiel eines Verfahrens zum Steuern von Anlagenkomponenten eines verfahrenstechnischen Anlagenmoduls bei einer Inbetriebnahme des Anlagenmoduls. Das Verfahren kann zumindest teilweise computerimplementiert sein.

Beispielsweise wird eine Wasserfahrt zur Inbetriebnahme des Anlagenmoduls durchgeführt. Im ersten Schritt S1 des Verfahrens wird ein Sensorwert einer physikalischen Größe, wie z.B. ein Durchflusswert oder ein Füllstandwert, eines ersten chemischen Stoffs in einer Anlagenkomponente des verfahrenstechnischen Anlagenmoduls erfasst. Vorzugsweise ist der erste chemische Stoff chemisch inert und wird lediglich für eine Inbetriebnahme des verfahrenstechnischen Anlagenmoduls genutzt. Der erste chemische Stoff wird in einem in den Anlagenkomponenten des verfahrenstechnischen Anlagenmoduls ablaufenden verfahrenstechnischen Prozess geändert; beispielsweise erfolgt lediglich eine Temperaturänderung, aber keine chemische Reaktion.

Im zweiten Verfahrensschritt S2 wird ein Teil des verfahrenstechnischen Prozesses, der in den Anlagenkomponenten ablaufen soll, parallel zur Wasserfahrt computergestützt simuliert. Folglich läuft der zweite Verfahrensschritt S2 vorzugsweise parallel und in Echtzeit zum ersten Verfahrensschritt S1 ab. Dabei wird eine chemische Reaktion, eine Temperaturänderung und/oder ein Stofftransport des verfahrenstechnischen Prozesses in mindestens einem Teil der Anlagenkomponenten für mindestens einen zweiten chemischen Stoff/für zweite chemische Stoffe in Abhängigkeit des Sensorwerts simuliert und ein simulierter Temperaturwert und ein simulierter Stoffmengenanteil des zweiten chemischen Stoffs ermittelt. Der zweite chemische Stoff unterscheidet sich vorzugsweise vom ersten chemischen Stoff und entspricht vorzugsweise demjenigen chemischen Stoff, mit dem das verfahrenstechnische Anlagenmodul nach der Inbetriebnahme betrieben werden soll. Der zweite chemische Stoff kann auch ein Stoffgemisch sein.

Im nächsten Verfahrensschritt S3 werden der simulierte Temperaturwert und der simulierte Stoffmengenanteil des zweiten chemischen Stoffs/ der zweiten chemischen Stoffe ausgegeben.

Im nächsten Verfahrensschritt S4 werden Aktoren der Anlagenkomponenten in Abhängigkeit des simulierten Temperaturwerts, des simulierten Stoffmengenanteils und des erfassten Sensorwert gesteuert. Der zweite bis vierte Verfahrensschritt S2-S4 können insbesondere iterativ durchgeführt werden, wobei Steuerbefehle zum Steuern der Aktoren der Anlagenkomponenten bei der Simulation in Verfahrensschritt S2 berücksichtigt werden.

Figur 4 zeigt ein Ausführungsbeispiel eines Verfahrens zum Steuern von Anlagenkomponenten eines verfahrenstechnischen Anlagenmoduls bei einem Betrieb des Anlagenmoduls. Dazu wird der real ablaufende verfahrenstechnische Prozess zumindest zum Teil während des Betriebs der Anlage parallel computergestützt simuliert.

Im ersten Verfahrensschritt S1 des Verfahrens wird ein Sensorwert, wie z.B. ein Durchflusswert, eines chemischen Stoffs in einer Anlagenkomponenten des verfahrenstechnischen Anlagenmoduls erfasst. Der chemische Stoff wird in einem in den Anlagenkomponenten des verfahrenstechnischen Anlagenmoduls ablaufenden verfahrenstechnischen Prozess geändert.

Im zweiten Verfahrensschritt S2 wird der verfahrenstechnische Prozess computergestützt parallel zum Betrieb simuliert. Dabei wird eine chemische Reaktion, eine Temperaturänderung und/oder ein Stofftransport des verfahrenstechnischen Prozesses des chemischen Stoffs in Abhängigkeit des gemessenen Sensorwerts simuliert und ein simulierter Temperaturwert und ein simulierter Stoffmengenanteil des chemischen Stoffs ermittelt.

Im nächsten Verfahrensschritt S3 werden der simulierte Temperaturwert und der simulierte Stoffmengenanteil des zweiten chemischen Stoffs ausgegeben.

Im nächsten Verfahrensschritt S4 werden Aktoren der Anlagenkomponenten in Abhängigkeit des simulierten Temperaturwerts, des simulierten Stoffmengenanteils und des erfassten Durchflusswertes gesteuert.

Alle beschriebenen und/oder gezeichneten Merkmale können im Rahmen der Erfindung vorteilhaft miteinander kombiniert werden. Die Erfindung ist nicht auf die beschriebenen Ausführungsbeispiele beschränkt.

## Patentansprüche

1. Verfahrenstechnisches Anlagenmodul (AM) umfassend:
a. Anlagenkomponenten (K), die dazu eingerichtet sind, einen verfahrenstechnischen Prozess durchzuführen, wobei ein erster chemischer Stoff (CS1) in dem verfahrenstechnischen Prozess geändert wird,
b. einen Sensor (FM), der derart eingerichtet ist, einen Sensorwert einer physikalischen Größe des ersten chemischen Stoffs in einer Anlagenkomponente zu erfassen,
c. eine Simulationseinheit (SIM), die derart eingerichtet ist,
- eine chemische Reaktion, eine Temperaturänderung und/oder einen Stofftransport des verfahrenstechnischen Prozesses für einen zweiten chemischen Stoff (CS2) in Abhängigkeit des Sensorwerts computergestützt zu simulieren, wobei der erste chemische Stoff (CS1) und der zweite chemische Stoff (CS2) übereinstimmen oder sich unterscheiden,
und
- einen simulierten Temperaturwert (T) und einen simulierten Stoffmengenanteil (x) des zweiten chemischen Stoffs (CS2) auszugeben,
und
d. eine Steuereinheit (PLC), die derart eingerichtet ist, Aktoren der Anlagenkomponenten (K) in Abhängigkeit des simulierten Temperaturwerts (T), des simulierten Stoffmengenanteils (x) und des erfassten Sensorwerts (Q) zu steuern.

2. Verfahrenstechnisches Anlagenmodul nach Anspruch 1, wobei der erste chemische Stoff (CS1) chemisch inert ist.

3. Verfahrenstechnisches Anlagenmodul nach Anspruch 1 oder 2, wobei der Sensor (FM) ein Durchflusssensor oder ein Füllstandsensor ist.

4. Verfahrenstechnisches Anlagenmodul nach einem der vorhergehenden Ansprüche, wobei der Sensor (FM) als virtueller Sensor ausgestaltet ist und einen simulierten Sensorwert bereitstellt.

5. Verfahrenstechnisches Anlagenmodul nach einem der vorhergehenden Ansprüche, wobei Steuerbefehle (CTL) der Steuereinheit (PLC) bei der computergestützten Simulation des verfahrenstechnischen Prozesses berücksichtigt werden.

6. Verfahrenstechnisches Anlagenmodul nach einem der vorhergehenden Ansprüche, wobei die computergestützte Simulation in Echtzeit und parallel zum verfahrenstechnischen Prozess durchgeführt wird.

7. Verfahrenstechnische Anlage (SYS) umfassend mindestens zwei verfahrenstechnische Anlagenmodule (AM1, AM2, AM3) nach den Ansprüchen 1 bis 6 sowie eine übergeordnete Steuereinheit (POL), wobei die jeweiligen Steuereinheiten (PLC1, PLC2, PLC3) der verfahrenstechnischen Anlagenmodule (AM1, AM2, AM3) durch die übergeordnete Steuereinheit (POL) miteinander gekoppelt werden und wobei die jeweiligen Simulationseinheiten (SIM1, SIM2, SIM3) der verfahrenstechnischen Anlagenmodule (AM1, AM2, AM3) miteinander gekoppelt werden.

8. Verfahrenstechnische Anlage nach Anspruch 7, wobei die jeweiligen Simulationseinheiten (SIM1, SIM2, SIM3) der verfahrenstechnischen Anlagenmodule mittels einer Co-Simulationsumgebung (CSIM) miteinander gekoppelt werden und wobei ein von der ersten Simulationseinheit (SIM1) ausgegebener erster simulierter Temperaturwert (T1) und ein erster simulierter Stoffmengenanteil (x1) als Eingabedaten für die zweite Simulationseinheit (SIM2) genutzt werden.

9. Verfahren zum Steuern von Anlagenkomponenten eines verfahrenstechnischen Anlagenmoduls, mit den Verfahrensschritten:
a. Erfassen (S1) eines Sensorwerts einer physikalischen Größe eines ersten chemischen Stoffs (CS1) in einer Anlagenkomponenten (K) des verfahrenstechnischen Anlagenmoduls (AM), wobei der erste chemische Stoff (CS1) in einem in den Anlagenkomponenten des verfahrenstechnischen Anlagenmoduls ablaufenden verfahrenstechnischen Prozess geändert wird,
b. computergestütztes Simulieren (S2) einer chemischen Reaktion, einer Temperaturänderung und/oder eines Stofftransports des verfahrenstechnischen Prozesses für einen zweiten chemischen Stoff (CS2) in Abhängigkeit des Sensorwerts (Q), wobei der erste chemische Stoff (CS1) und der zweite chemische Stoff (CS2) übereinstimmen oder sich unterscheiden,
c. Ausgeben (S3) eines simulierten Temperaturwerts und eines simulierten Stoffmengenanteils des zweiten chemischen Stoffs, und
d. Steuern (S4) von Aktoren der Anlagenkomponenten in Abhängigkeit des simulierten Temperaturwerts, des simulierten Stoffmengenanteils und des erfassten Sensorwerts.

10. Computerprogrammprodukt, das direkt in einen programmierbaren Computer ladbar ist, umfassend Programmcodeteile, die dazu geeignet sind, die Schritte des Verfahrens gemäß Anspruch 9 durchzuführen.
